# EUROPEAN PATENT SPECIFICATION

(11) **EP 3 503 962 B1**
(45) Date of publication and mention of the grant of the patent: **26.02.2025**
(21) Application number: 17765257.5
(22) Date of filing: 11.08.2017
(51) Int. Cl.: A61N 1/04, A61N 1/22, A61N 1/36

(54) **DEVICE FOR THE TREATMENT OF CONSCIOUSNESS DISORDERS**
VORRICHTUNG ZUR BEHANDLUNG VON BEWUSSTSEINSSTÖRUNGEN
DISPOSITIF DE TRAITEMENT DES TROUBLES DE LA CONSCIENCE

(30) Priority: 29.08.2016 US 201615250431
(43) Date of publication of application: 03.07.2019
(73) Proprietor: CEFALY Technology Sprl, 4000 Liège (BE)
(72) Inventor: RIGAUX, Pierre, 4102 Seraing (BE)
(74) Representative: Brantsandpatents bv
(86) International application number: PCT/IB2017/054905
(87) International publication number: WO 2018/042278

(56) References cited:
- WO-A1-2013/022840
- WO-A2-2008/005478
- WO-A2-2009/137683
- CN-A- 102 698 360
- US-B1- 8 583 238
- ANGELAKIS EFTHYMIOS ET AL: "Transcranial Direct Current Stimulation Effects in Disorders of Consciousness", ARCHIVES OF PHYSICAL MEDICINE AND REHABILITATION, W.B. SAUNDERS, UNITED STATES, vol. 95, no. 2, 11 September 2013 (2013-09-11), pages 283 - 289, XP028815057, ISSN: 0003-9993, DOI: 10.1016/J.APMR.2013.09.002

## Description

### Field of the invention

The present disclosure pertains to a non-invasive device and method for the treatment of consciousness disorders using transcranial direct current stimulation (tDCS). The present invention is set out in the appended claims.

### Background

In recent years, resuscitation techniques have led to a considerable increase in the number of patients who survive severe brain injuries. Some patients recover in the first days after the injury while others die quickly. Others, however, recover more slowly through different stages before fully or partially recovering consciousness. Patients having consciousness disorders present major challenges concerning the diagnosis, prognosis, daily care and treatment.

Disorders of consciousness include coma and the vegetative state (VS). Patients in coma have complete failure of the arousal system with no spontaneous eye opening and are unable to be awakened by application of vigorous sensory stimulation. VS is characterized by the complete absence of behavioral evidence for self or environmental awareness. There is preserved capacity for spontaneous or stimulus-induced arousal, evidenced by sleep-wake cycles.

Some patients with severe alteration in consciousness have neurologic findings that do not meet criteria for VS. These patients demonstrate discernible behavioral evidence of consciousness but remain unable to reproduce this behavior consistently. This condition is referred to here as the minimally conscious state (MCS). MCS is distinguished from VS by the partial preservation of conscious awareness.

Non-invasive interventions have been used to improve the functional level of MSC patients. Said interventions are based on neuromodulation which includes the broad category of "transdermal electrical stimulation," referring to the electrical stimulation of the nervous system (brain, cranial nerves, peripheral nerves, etc.) through a subject's skin. Specific examples of transdermal electric stimulation (hereinafter "TES") may include transcranial stimulation, for example, through scalp electrodes and have been used to affect brain function in humans in the form of transcranial alternating current stimulation, transcranial direct current stimulation (hereinafter "tDCS"), cranial electrotherapy stimulation, and transcranial random noise stimulation.

Despite several research and clinical trials on non-pharmacological treatments, there is currently no effective standardized treatment and no evidence-based treatment for patients suffering from consciousness disorder, in particular the minimal consciousness state (MCS). Documents WO 2009/137683 and WO 2013/022840 disclose known prior art devices.

The aim of the present invention is to provide a solution to overcome at least part of the above mentioned disadvantages. The invention thereto provides a treatment device as described below and as defined by the appended claims.

### SUMMARY

The invention provides a device for the treatment of consciousness disorder, according to claim 1.

Use of the device according to the invention provides several advantages compared to the methods of the prior art. It is safe, non-invasive and drug free. Additionally, it is highly efficient as the consciousness level of treated patients has been considerably improved which leads to a better life quality thanks to the possible communication with family members, for instance. The device according to the invention can also be used outside of medical facilities, e.g. at home by relatives, as a daily care.

### DESCRIPTION OF THE FIGURES

**FIG. 1** shows the time scale of the applied treatment and the CRS-R measurements.

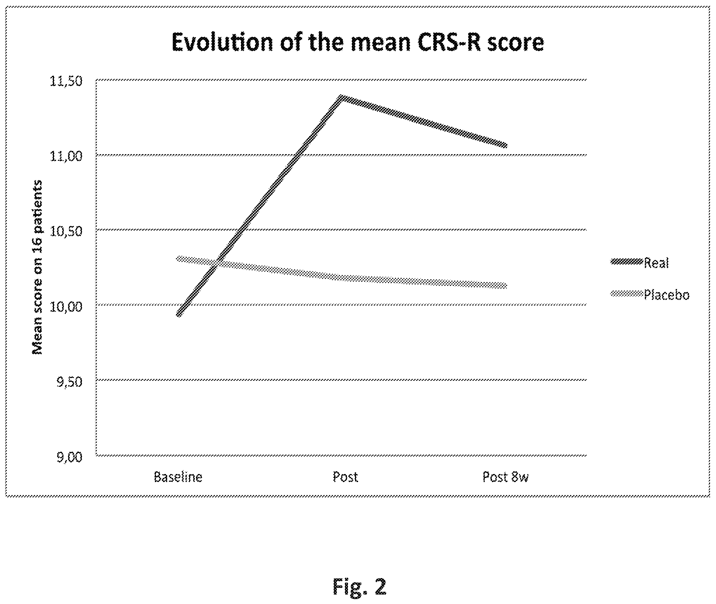

**FIGS. 2** **and** **9** show the effect of a method according to the description.
**FIG. 3** shows the electrode location on the head of a patient in order to stimulate the left dorso-lateral prefrontal cortex.
**FIGS. 4 to 6** show several aspects of a head-mountable device for the treatment of consciousness disorder.
**FIG. 7** shows various components of a power source for use in the head-mountable device of FIGS. 4 to 6.
**FIG. 8** shows a printed circuit board as used in the power source of FIG. 7.

### DETAILED DESCRIPTION OF THE INVENTION

The present invention relates to a non-invasive device for the treatment of consciousness disorder via transcranial direct current stimulation.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

### Method

The present description provides a non-claimed method for the treatment of consciousness disorder comprising the steps of:
a) positioning at least two electrodes on a patient's head such that the first electrode is positioned on the right supraorbicular cortex and the second electrode is positioned on the left dorso-lateral prefrontal cortex;
b) connecting said electrodes to at least one source of electrical current;
c) delivering direct current to the electrodes, and
d) removing the electrodes from the patient's head after a time T;
wherein steps a) to d) represent a treatment session and wherein at least one treatment session is applied on the patient's head at least 2 days per week.

In a preferred embodiment, one treatment session is applied at least one time every 24 hours 7 days per week, preferably for maximum 6 days per week, more preferably for maximum 5 days per week. The treatment sessions can be applied at any time of the day and are preferably applied in the morning so between 6 am and 12 am. In a preferred embodiment, the repetitive treatment sessions are applied to the patient at the same time. For instance, if the first session has been applied on Monday at 10 am, the next session is to be applied on Tuesday at 10 am.

Preferably, the time T in step d) is at least 5 minutes and at most 30 minutes, preferably at least 10 min and at most 20 min.

The intensity of the direct current is of from 0.5 to 3mA, preferably of from 1 to 2.5mA, more preferably about 2mA.

Preferably, the intensity of the direct current increases progressively following a slope of from 0.06mA/sec to 0.6mA/sec, more preferably about 0.4 mA/sec. The intensity of the direct current increases progressively during a period of time T' wherein T' is at least 1 second and at most 5 minutes, preferably T' is about 5 seconds.

Preferably the density of the direct current is of from 0 to 100µA/cm², preferably of from 50 to 60µA/cm².

The treatment is applied during at least one week and at most 10 weeks, preferably at least at least 2 weeks and at most 9 weeks, more preferably at least 3 weeks and at most 8 weeks, even more preferably at least 4 weeks and at most 7 weeks, most preferably at least 5 weeks and at most 6 weeks. The treatment can also be applied for several months or several years.

### Example 1: Preliminary clinical trial

### - Patients

A group of traumatic and non-traumatic patients in a sub-acute and chronic MSC for at least 3 months has been selected for the treatment. The group consisted of 16 patients and comprised a mix of male and female patients of different age but of at least 18 years old. Patients with a metallic cerebral implant or a pacemaker were excluded from the group. Also patients who received a drug treatment and/or suffering from other disorders, such as epilepsy, were excluded from the group.

### - Treatment

One treatment session consisted of positioning two electrodes on the skull of the patient. The cathode was positioned above the right eyebrows and more exactly on the right supraorbicular cortex (FP2). The anode was positioned on the left dorso-lateral prefrontal cortex (F3). The electrodes were connected to an electric current generator. Direct current having an intensity of 2mA and a density of from 50 to 60µA/cm² was delivered to the patients for 20 min. The increase in current intensity was gradual following a slope of 0.4mA/s.

The above described treatment session has been repeated every day at the same time for 5 days per week.

Each patient received, in a random order, the above treatment for 4 weeks and a placebo treatment for 4 weeks. The placebo treatment was made of a similar setup as the real treatment, but the stimulation was only active for 5 seconds. For the rest of the 20 minutes, no direct current was delivered by the placebo treatment. The two 4-week treatments were separated by a 8-week period without treatment. The treatment was applied either by the family at home, or by a nurse in a rehab center. The caregiver was blinded on the treatment.

### - Results measurement and analysis

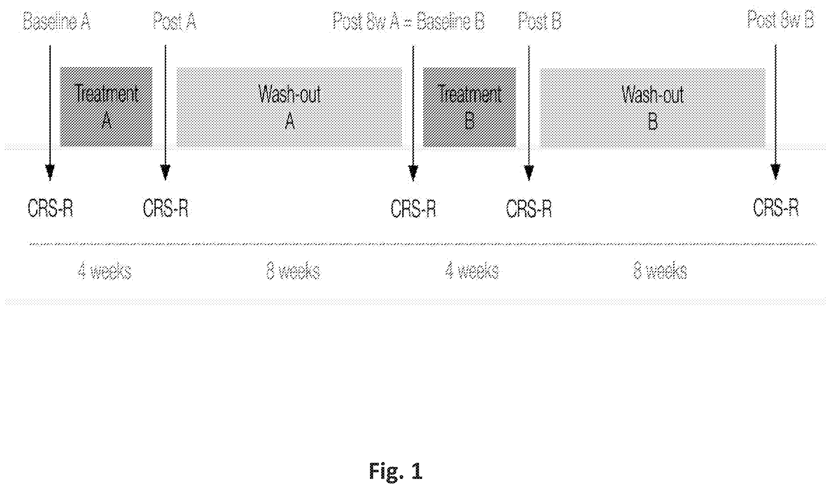

Treatment result was assessed based on the Coma Recovery Scale Revised (CRS-R) as described in Giacino et al., 2004, Arch Phys Med Rehabil*.* The measurements were performed on each patient before the treatment (Baseline A, figure 1), immediately after the treatment A (Post A, Figure 1), 8 weeks after the end of the treatment A, which corresponds to the beginning of treatment B (Post 8w A = Baseline B, Figure 1), immediately after the treatment B (Post B, Figure 1) and 8 weeks after the end of treatment B (Post 8w B, Figure 1). As mentioned earlier, patients were randomly assigned either real treatment or placebo treatment as treatment A, and the other as treatment B.

The mean delta between Baseline and Post CRS-R score is +1.44±2.19 (statistically significant, *p*<0.05) for the real treatment and -0.13±2.25 (*p*>0.80) for the placebo treatment. The difference of this delta between the real treatment and the placebo treatment is also statistically significant (*p*<0.05).

At the individual level, 10 out of 16 patients, i.e. 62.5% of the patients, showed a tDCS-related improvement. This is better than with a single session where the improvement observed in only 43% of the patients (reported in Thibaut et al. 2014). Repeating the stimulation increases patient's reactivity to the treatment.

### Device

The invention provides a device as defined in the appended claims for the treatment of consciousness disorder via tDCS. The device comprises a first electrode, a second electrode, a positioning means and a power source. The positioning means is adapted for positioning the first electrode on the right supraorbital region of a patient's head, and in particular for stimulating the right supraorbicular cortex (FP2). The positioning means is furthermore adapted for positioning the second electrode on a second region on the patient's head, whereby the second region corresponds to the left dorso-lateral prefrontal cortex (F3). The positioning means is also adapted for attaching the power source to the positioning means. The positioning means furthermore comprises at least one electrical conductor, and preferably at least two electrical conductors, for transmitting direct current from the power source to each of the electrodes.

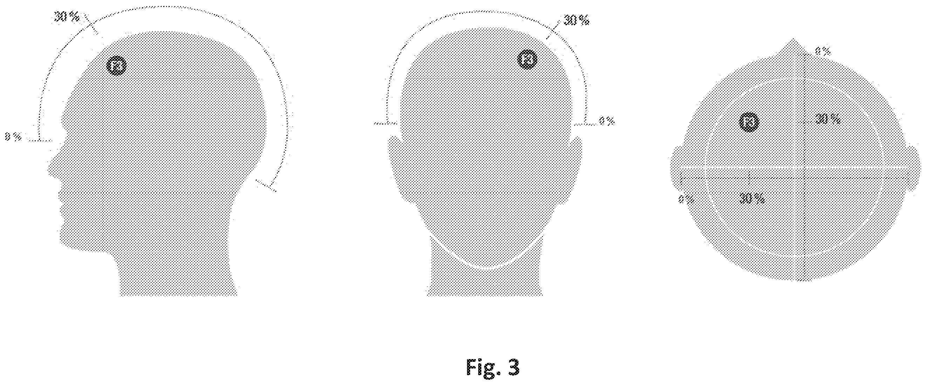

Figure 3 shows a schematic representation of the location of the second region on a patient's head, corresponding to the left dorso-lateral prefrontal cortex (F3). The second region is located on about 30% of the distance from the nose to the occiput; and on about 30% of the distance from the left ear to the right ear. The right supraorbital region or the region corresponding to the right supraorbicular cortex (FP2) is located just above the right eyebrow.

In a preferred embodiment, the first electrode (right supraorbital region; FP2) is a cathode and the second electrode (left dorso-lateral prefrontal cortex; F3) an anode. The anode may reduce the resting membrane threshold, thereby stimulating neuronal depolarization, while the cathode has opposite effects. Anodal stimulation may modulate long-term potentiation by enhancing NMDA receptor synaptic activity, while cathodal stimulation may reduce it.

In a preferred embodiment, the entire device is head-mountable, i.e. both the positioning means as well as the power source can be mounted on the patient's head. This is advantageous as it requires limited space and allows for easy repositioning of the patient's head, without having to interrupt a treatment session or getting tangled in wiring.

In a preferred embodiment, the power source is adapted for providing a direct current to the electrodes comprising an amplitude of at least 0.5 mA and at most 8 mA, preferably at least 1.0 mA and at most 4 mA, more preferably at least 1.5 mA and at most 3 mA, such as an amplitude of about 1.5 mA, about 1.6 mA, about 1.7 mA, about 1.8 mA, about 1.9 mA, about 2 mA, about 2.1 mA, about 2.2 mA, about 2.3 mA, about 2.4 mA, about 2.5 mA, about 2.6 mA, about 2.7 mA, about 2.8 mA, about 2.9 mA or about 3.0 mA. In a most preferred embodiment, the power source is adapted for providing a direct current to the electrodes comprising an amplitude of about 2 mA.

In a preferred embodiment, the power source is adapted for providing a direct current during a treatment time Tₜ of at least 5 minutes, preferably at least 10 minutes, most preferably at least 15 minutes, such as about 15 minutes, about 16 minutes, about 17 minutes, about 18 minutes, about 19 minutes, about 20 minutes, about 21 minutes, about 22 minutes, about 23 minutes, about 24 minutes, about 25 minutes, about 26 minutes, about 27 minutes, about 28 minutes, about 29 minutes or about 30 minutes. In a most preferred embodiment, the power source is adapted for providing a direct current during a treatment time Tₜ of about 20 minutes.

In a preferred embodiment, the power source is adapted for providing a direct current during a treatment time Tₜ of at most 180 minutes, preferably at most 150 minutes, more preferably at most 120 minutes, even more preferably at most 90 minutes, even more preferably still at most 75 minutes, yet more preferably at most 60 minutes, yet even more preferably at most 45 minutes, most preferably at most 30 minutes, such as about 30 minutes, about 29 minutes, about 28 minutes, about 27 minutes, about 26 minutes, about 25 minutes, about 24 minutes, about 23 minutes, about 22 minutes, about 21 minutes, about 20 minutes, about 19 minutes, about 18 minutes, about 17 minutes, about 16 minutes or about 15 minutes. In a most preferred embodiment, the power source is adapted for providing a direct current during a treatment time Tₜ of about 20 minutes.

In a preferred embodiment, the power source is adapted to increase the direct current progressively with a slope of at least 0.06 mA/s and at most 0.6 mA/s, preferably a slope of about 0.4 mA/s. Said progressive increase of said direct current is performed for a time span T' required to increase the direct current from 0 mA to the desired value. For example, for a desired direct current amplitude of about 2 mA and a slope of about 0.4 mA/s, said progressive increase is performed during a time span T' of about 5 seconds. T' is preferably at least 1 second and at most 5 minutes.

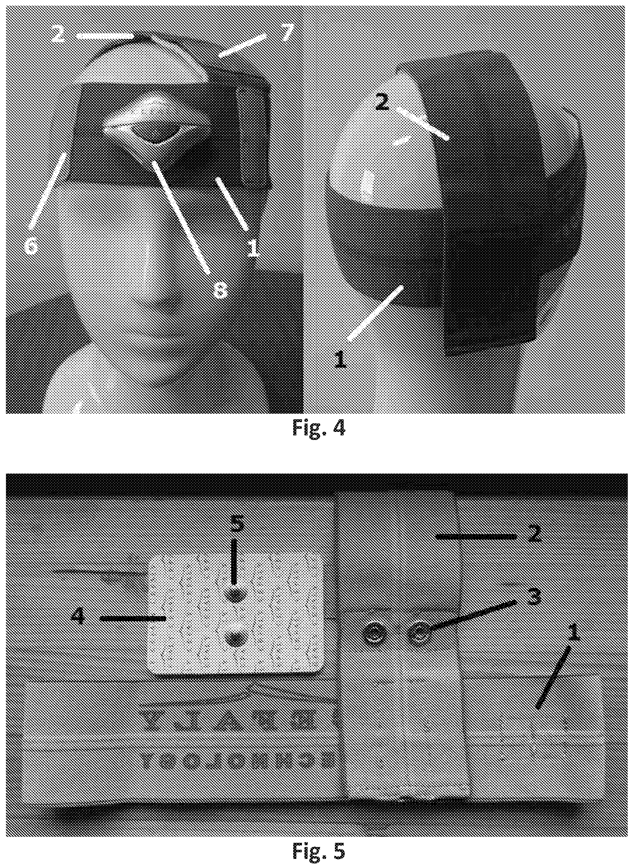

Figures 4 to 8 show further preferential embodiments of the device. In a preferred embodiment, the positioning means comprises a headband (1) for placement around the patient's head and a transversal band (2). The headband (1) is adapted for positioning the first electrode on the right supraorbital region (6; FP2), just above the right eyebrow. The transversal band (1) is attached to the headband and is adapted for positioning the second electrode (4) on the second region corresponding to the left dorso-lateral prefrontal cortex (7; F3). Preferably, the headband (1) and the transversal band (2) comprise elastic fabric. The at least one electrical conductor, e.g. wire or conductive fabric, is thereby expandable to adjust to movement of the bands. Preferably, the transversal band (2) comprises a first end portion fixedly attached to the headband (1). Preferably, the transversal band (2) comprises a second end portion opposite the first end portion, whereby the device comprises a hook-and-loop fastener (10) for attaching the second end portion to the headband (1). Preferably, the headband comprises a designated portion (9) to which the power source (8) may be attached, whereby the (location of the) designated portion (9) is configured for correct positioning of the first and the second electrode upon positioning said designated portion (9) centrally on the patient's forehead. The headband (1) and the transversal band (2) may comprise a width of at least 55 mm. The headband (1) may comprise a diameter of minimally 160 mm or a length of about 500 mm (≈ 160 mm x n) in unexpanded state. The transversal band may comprise a length of at least 380 mm. The hook-and-loop fastener may comprise a rectangular hook patch and a rectangular loop patch, each of about 70 mm by about 55 mm.

This is advantageous as it allows to easily and correctly place the device and therefore the electrodes on a patient's head. The elasticity of the bands (1, 2) allows for adjustment to various head sizes, according to the one size fits all (or most) principle. First, the headband (1) plus first electrode may be mounted around the head of the patient with said designated portion centrally on the forehead. Then, the transversal band (2) plus second electrode may be placed over the top of the patient's head, thereby positioning the second electrode on the second region (corresponding to the left dorso-lateral prefrontal cortex (F3)), by attaching the hook-and-loop fastener (10). Subsequently, the power source (8) may be attached to the designated portion (9) of the headband (1).

In a preferred embodiment, each of the first and second electrode (4) is attachable to the positioning means (1, 2) via at least one snap button (3, 5), preferably via at least two snap buttons (3, 5), i.e. the device comprises in total at least two, and preferably at least four, snap buttons to attach the first and the second electrode to the positioning means. The snap buttons also provide electrical contact between an electrical conductor and an electrode. At least two snap buttons per electrode are advantageous as it prevents rotation of the electrode. Preferably, the female parts of the snap buttons are fixedly attached to the positioning means (headband; transversal band) and the male parts of the snap buttons to the electrodes. For two snap buttons per electrode, the female parts are positioned along the width of the band (headband; transversal band).

In a preferred embodiment, the power source (8) is attachable to the positioning means via at least one magnet, preferably via at least two magnets. The magnets may also provide electrical contact between the electrical conductors and the power source. At least two magnets are advantageous as it prevents rotation of the power source. In addition, two electrical contacts are required with the power source in order to provide said direct current. The magnets are preferably positioned within the designated portion (9) of the headband (1). Alternatively or in addition, the power source may also be attached to the positioning means by snap buttons.

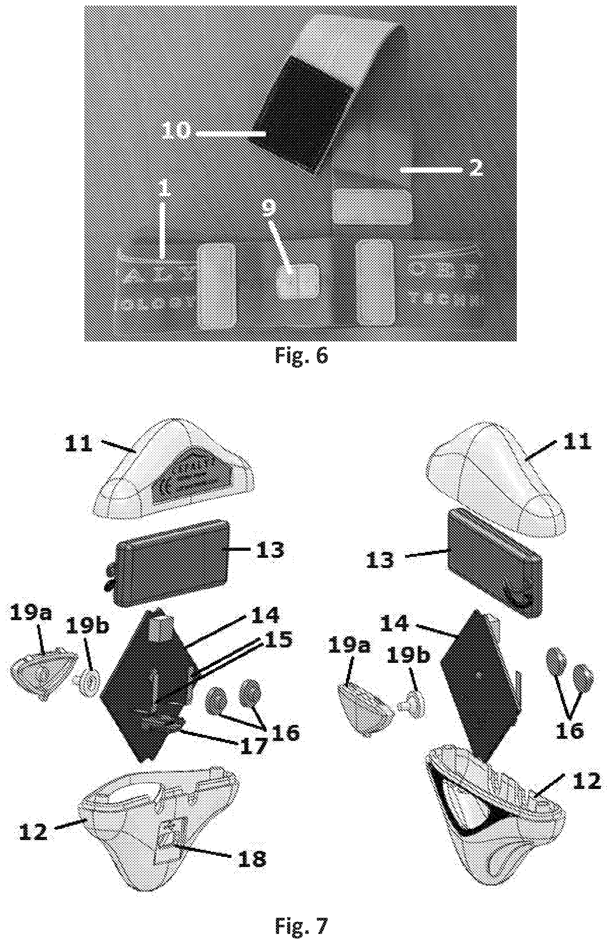

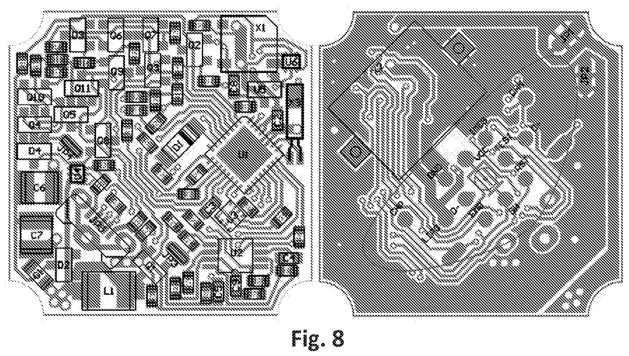

At least some of the contents of the power source are shown in Figures 7 and 8. The power source comprises a bottom cover (12) and a top cover (11). The covers are preferably made in polycarbonate by injection molding. The bottom cover may comprise two pin reception holes and the top cover may comprise two pins, the top and bottom cover thereby being mutually attachable by inserting the pins in the pin reception holes. The power source, and more in particular the bottom cover, may comprise an opening (18) for a micro USB connector (17). The power source may comprise a rechargeable battery (13), such as a Lithium battery, which may be recharged via said USB connector (17). The power source may furthermore comprise a printed circuit board (PCB) comprising a base plate (14; Figure 8), said USB connector (17), and two electrical contact blades (15) which may be placed in electrical contact with said designated portion (9) of said headband (1) via two intermediary conductive contacts (16) in one or both of said covers. The battery (13) comprises connection wires which are welded to the base plate (14) of the PCB. The rechargeable battery (13) may be placed in between the base plate (14) of the PCB and the electrical contact blades (15). The power source may furthermore comprise a user button (19a) and a user button transmitter (19b) for mechanically connecting the user button (19a) with the base plate (14). The PCB may comprise a micro controller unit (MCU) or microprocessor, rendering the power source a clocked digital device. The power source may comprise one or multiple LEDs for indicating usage status, battery status, recharging status, and the like. The power source may comprise a tangible non-transitory computer-readable storage medium, such as flash memory. The PCB may comprise an internal world clock. The power source may be configured for storing usage data on the storage medium. The usage data may comprise a world time stamp and a treatment duration. The usage data may comprise further information, such as, for example, the direct current amplitude. The stored usage data may be retrieved with an external computing device via said USB connector. New computer-readable instructions (operating code) and/or new operating settings (e.g. treatment time Tₜ; direct current amplitude) may also be uploaded from an external computing device to the storage medium via said USB connector. The PCB base plate (14) may comprise as dimensions about 2.7 mm by about 2.7 mm and a thickness of about 0.8 mm. The power source may comprise as dimensions about 55 mm by about 40 mm by about 15 mm.

In a preferred embodiment, the current density provided via said electrodes is at most 0.1 mA/cm² in order to prevent electrochemical burns. Preferably, the current density provided via said electrodes is at least 30 µA/cm² and at most 60 µA/cm², such as a value of about 45 µA/cm². For a direct current of about 2 mA, an electrode surface of at least 20 cm² is required to obtain a current density of at most 100 µA/cm². The electrodes may be rectangular comprising the dimensions 80 mm by 55 mm, which for a current of about 2 mA corresponds to a current density of about 45.5 µA/cm². The electrodes may be rectangular comprising the dimensions 70 mm by 50 mm, which for a current of about 2 mA corresponds to a current density of about 57.1 µA/cm².

In an embodiment, an electrode may comprise a sponge. The sponge may be impregnated with salt water prior to attachment to the positioning means (headband, transversal band). The impedance of the direct current circuit is preferably below 10 kΩ. The maximum output voltage of the power source is preferably below 26 V. For a direct current amplitude of 2 mA and an impedance of at most 10 kΩ, a voltage of at most 20 V is required.

One of ordinary skill in the art will appreciate that aspects discussed above in conjunction with the device also concern the treatment method.

### Example 2: Extended clinical trial

### - Patients

Inclusion criteria were patients over 16 years old, in stable vital condition (e.g.: no infection, intubation, recent hospitalization) and in MCS for more than three months post injury. The diagnosis of MCS was established based on at least 5 successive behavioral assessments (using the CRS-R) within a two weeks period. Patients with metallic cerebral implants, cerebral shunts or pacemakers were excluded in agreement with the tDCS safety guidelines for human beings. Patients with frontal craniectomy or cranioplasty, NMDA receptor inhibitors drugs (e.g. dextromethorphan) or premorbid neurological disorders were not included. We also excluded patients who received tDCS, or other non-invasive brain stimulation (NIBS), less than 3 months before their inclusion into this study.

Patients were considered as drop-outs if one of the following scenarios occurred:
1. introduction of a new central-acting drug (e.g., antiepileptic or sedative drugs, amantadine or intrathecal baclofen);
2. change in rehabilitation (increase or reduction of a minimum of two sessions per week);
3. surgical intervention;
4. infection with deterioration of patients' general condition or an occurrence of an important medical event (e.g., sign of pain, seizure); and
5. if less than 80% of the active treatment sessions were applied in accordance to the protocol.

In total, 37 patients were assigned to receive both anodal and sham tDCS in a crossover study between June 2014 and January 2017. Ten patients were excluded from the study due to one of the abovementioned exclusion criteria. The 10 drop-outs did not differ from the sample in terms of age (t=-0.624; p=0.540), time since injury (t=0.836; p=0.410) or baseline CRS-R (t=-1.470; p=0.151). Out of these 27 patients, 5 patients did not and 22 patient did receive at least 80% (i.e., ≥ 16 out of 20 sessions) of the active treatment sessions.

To monitor the compliance in a remote setting, the device recorded the stimulation sessions, which provided a feedback to the investigators regarding the time of stimulation and amount of completed sessions, once the study protocol was completed.

### - Treatment

Caregivers were trained and were evaluated on the device use. Each patient received both anodal and sham tDCS stimulations in a crossover, randomized order. A set of two devices was assigned to each patient. One device was programmed to deliver anodal (active) tDCS, while the other was sham tDCS. Anodal parameters consisted of 2 mA amplitude during 20 minutes treatment time. Sham parameters consisted of a 5-second 2 mA ramp up period, followed by a 5-second 2 mA stimulation period, followed by a 5-second ramp down procedure to mimic the somatosensory effect of active tDCS. A randomization sequence assigned in a 1:1 ratio the first device to deliver either active or sham stimulation. The investigators and patients were blinded to the allocation. Direct current was applied by a battery-driven constant current stimulator using saline-soaked surface sponge electrodes (7 cm by 5 cm) with the anode positioned over the left dorso-lateral prefrontal cortex and the cathode placed over the right supraorbital region. tDCS was performed daily by the same person, at the same time of the day, for 4 weeks, 5 times a week (twenty stimulation sessions per period - the two periods were separated by 8 weeks of washout). CRS-R assessments were performed at baseline, week 4 (after the end of the stimulation period) and week 12 (after 8 weeks of washout) for each stimulation period (active and sham). The CRS-R consists of 23 hierarchically arranged items, comprised of 6 subscales assessing auditory, visual, motor, verbal, communication and arousal functions. CRS-R was assessed by trained and experienced blinded assessors.

### - Statistical analysis

Statistical analysis was performed using Stata (StataCorp. 2013. Stata Statistical Software: Release 13. College Station, TX: StataCorp LP). Baseline characteristics between groups (active-sham and sham-active) were tested for comparability using Student's t-test for continuous variables and the Chi square test for dichotomous variables.

The carry-over effect was investigated with a Wilcoxon match-paired signed-rank test comparing the CRS-R total scores of the two baselines. If no carry-over effect was found, the treatment effect was then assessed using a Wilcoxon match-paired signed-rank test. The treatment effect per group at 4 weeks of treatment and at 8 weeks of follow-up was analyzed using the differences in CRS-R total score (i.e. delta), between week 4 - baseline and week 12 - baseline, respectively. Statistical analysis was performed for the patients who completed the entire study protocol (27 patients) and for patients who received at least 80% of the active treatment sessions as per exclusion criteria (22 patients). Results were considered significant at p<0.05. The effect size (Cohen's d effect size) was calculated from the difference in means and standard deviations between baseline and post-treatment comparing active with sham tDCS.

The correlation between CRS-R improvement (i.e.: delta week 4 - baseline) and the time since injury was calculated using a Spearman correlation test. The treatment effect between etiologies (i.e., traumatic versus non-traumatic for active and sham intervention - 4 groups) was compared with a Kruskal-Wallis rank test.

### - Result and analysis

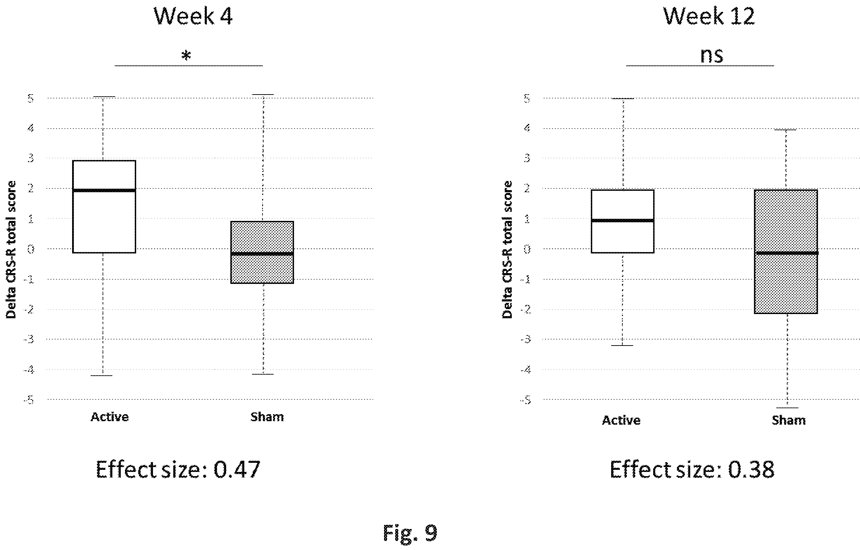

27 patients completed the study and 22 patients received at least 80% of the stimulation sessions. The patients adequately tolerated all the tDCS sessions. The adherence (i.e.: percentage of treatment sessions performed) was 94±14%. Regarding the changes in the CRS-R total score, no carry-over effect was observed (n=27: Z=1.503, p=0.132; n=22: Z=0.893; p=0.372). A moderate effect size was observed at the end of the 4 weeks of tDCS in favor of the active treatment (n=27: effect size=0.47; p=0.053; n=22: effect size=0.53; p=0.043). No significant differences between groups' effect sizes were observed at 8-week follow-up, as shown in Figure 9.

The median CRS-R total scores for the completers only increased from 9 (6 - 12) at baseline to 10 (7 - 14) after 4 weeks, and decreased to 9 (8 - 13) at 8-week follow-up, for the active stimulation period. For the sham sessions, the median was 9 (7 - 12) at baseline, 9 (6 - 12) after 4 weeks of tDCS and 9 (6 - 12) at 8-weeks follow-up. For the 22 patients who received at least 80% of the stimulation sessions, the median CRS-R total scores increased from 9 (6 - 11) at baseline to 11 (7 - 14) after 4 weeks and decreased to 9.5 (8 - 13.5) at 8-week follow-up, for the active stimulation period. For the sham sessions, the median was 9.5 (7 - 12) at baseline, 10 (6 - 13) after 4 weeks of tDCS and 9 (6 - 12) at 8-weeks follow-up.

This suggests long-term and ongoing treatment of consciousness disorder, and in particular MCS.

No correlation between the time since injury and CRS-R improvement was found (rho=0.0213; p=0.878). Regarding the etiology, no difference between the four groups (active tDCS-traumatic, active tDCS-non-traumatic, sham tDCS-traumatic, sham tDCS-non-traumatic) was identified (chi-square=1.721; p=0.6322).

At the single subject level, a difference between the two groups (active versus sham) was identified for three items only, namely the object localization (present in 3 patients in the active group, while no patient in the sham group demonstrated this behavior), intentional communication (2 patients in the active group recovered this behavior after the active session, while no patient in the sham group) and object manipulation (present for 1 patient in the active group and none in the sham group).

### - Outlook

In order to further investigate the results of the treatment method, neurophysiological measurements such as electroencephalography (EEG), magnetic resonance imaging (MRI), and/or fluorodeoxyglucose positron emission tomography (PET) may be used in future studies to observe cortical and subcortical areas which are known to be involved in attention and working memory.

### Further medical uses

The description and examples above disclose the method and the device for treatment of consciousness disorder, and in particular the minimally conscious state (MCS).

The device and/or method based on transcranial direct current stimulation (tDCS) may in addition to treatment of disorders of consciousness (DOC), such as the vegetative state (VS), the unresponsive wakefulness syndrome (UWS) and the minimally conscious state (MCS), also be used for treatment of cognitive functions in case of multiple sclerosis, chronic pain, headaches, sleeping disorders, depression and stroke. The invention is set out in the claims that follow.

## Claims

1. A device for the treatment of consciousness disorder via transcranial direct
current stimulation, the device comprising a first electrode and a second electrode (4), a positioning means (1, 2) and a power source (8), the positioning means:
a. adapted for positioning the first electrode on the right supraorbital region (FP2) of a patient's head;
b. adapted for positioning the second electrode on a second region on the patient's head, the second region corresponding to the left dorso-lateral prefrontal cortex (F3);
c. comprising at least one electrical conductor for transmitting direct current from the power source to the electrodes,
wherein the positioning means comprises a designated portion (9) to which the power source (8) is configured to be attached, whereby the location of the designated portion (9) is configured for correct positioning of the first and the second electrode upon positioning said designated portion (9) centrally on the patient's forehead.

2. The device according to any one of the preceding claims, wherein the power source is adapted for providing a direct current to the electrodes comprising an amplitude of at least 0.5 mA and at most 8 mA.

3. The device according to any one of the preceding claims, wherein the power source is adapted for providing a direct current during a treatment time Tₜ of at least 5 minutes.

4. The device according to any one of the preceding claims, wherein the power source is adapted for providing a direct current during a treatment time Tₜ of at most 180 minutes.

5. The device according to any of the preceding claims, wherein the power source is adapted for providing a direct current to the electrodes comprising an amplitude of at least 1.6 mA and at most 2.4 mA, wherein the power source is further adapted for providing a direct current during a treatment time Tₜ of at least 15 minutes and at most 45 minutes.

6. The device according to any one of the preceding claims, wherein the first electrode is a cathode and wherein the second electrode is an anode.

7. The device according to any one of the preceding claims, wherein the positioning means comprises a headband for placement around the patient's head and a transversal band, the headband adapted for positioning the first electrode over the right supraorbital region, the transversal band attached to the headband and adapted for positioning the second electrode over the second region.

8. The device according to claim 7, wherein the power source is configured to be attached to the headband on a designated portion of the headband, corresponding to a central portion of the forehead of the patient.

9. The device according to any one of the preceding claims, wherein each of the first and the second electrode is configured to be attached to the positioning means via at least two snap buttons for providing electrical contact between an electrical conductor and the electrode and for preventing rotation of the electrode.

10. The device according to any one of the preceding claims, wherein the power source is configured to be attached to the positioning means via at least one magnet.

11. The device according to any one of the preceding claims, wherein the device comprises at least two electrical conductors for transmitting direct current from the power source to the electrodes, wherein the power source is configured to be attached to the positioning means via at least two magnets, and whereby the magnets also provide electrical contact between the electrical conductors and the power source.

12. The device according to any one of the preceding claims, wherein the device is adapted for providing via said electrodes a current density of at most 100 µA/cm².

13. The device according to any of the preceding claims, wherein the device is adapted for providing via said electrodes a current density between 50 and 60 µA/cm², and wherein the power source is adapted for providing a direct current and for increasing said direct current with a slope of at least 0.06 mA/s and at most 0.6 mA/s.

14. The device according to any of the preceding claims, wherein at least two magnets are adapted to prevent rotation of the power source.

15. The device according to any of the preceding claims, wherein at least one magnet is disposed on the positioning means to as to be mounted centrally on a forehead of the patient.

## Patentansprüche

1. Vorrichtung zur Behandlung einer Bewusstseinsstörung mittels transkranieller Stimulation mit Gleichstrom, wobei die Vorrichtung eine erste Elektrode und eine zweite Elektrode (4), ein Positionierungsmittel (1, 2) und eine Energiequelle (8) umfasst, wobei das Positionierungsmittel:
a. für das Positionieren der ersten Elektrode an der rechten supraorbitalen Region (FP2) des Kopfs eines Patienten eingerichtet ist,
b. für das Positionieren der zweiten Elektrode an einer zweiten Region des Kopfs des Patienten eingerichtet ist, wobei die zweite Region dem linken dorsolateralen präfrontalen Cortex (3) entspricht,
c. mindestens einen elektrischen Leiter zum Übertragen von Gleichstrom von der Energiequelle zu den Elektroden umfasst,
wobei das Positionierungsmittel einen gekennzeichneten Abschnitt (9) umfasst, an den angebracht zu werden die Energiequelle (8) gestaltet ist, wobei die Position des gekennzeichneten Abschnitts (9) für das korrekte Positionieren der ersten und der zweiten Elektrode mittig auf der Stirn des Patienten nach dem Positionieren des gekennzeichneten Abschnitts (9) gestaltet ist.

2. Vorrichtung nach einem der vorhergehenden Ansprüche, wobei die Energiequelle dafür eingerichtet ist, einen Gleichstrom für die Elektroden bereitzustellen, der eine Amplitude von mindestens 0,5 mA und höchstens 8 mA umfasst.

3. Vorrichtung nach einem der vorhergehenden Ansprüche, wobei die Energiequelle dafür eingerichtet ist, einen Gleichstrom während einer Behandlungszeit Tₜ von mindestens 5 Minuten bereitzustellen.

4. Vorrichtung nach einem der vorhergehenden Ansprüche, wobei die Energiequelle dafür eingerichtet ist, einen Gleichstrom während einer Behandlungszeit Tₜ von höchstens 180 Minuten bereitzustellen.

5. Vorrichtung nach einem der vorhergehenden Ansprüche, wobei die Energiequelle dafür eingerichtet ist, einen Gleichstrom für die Elektroden bereitzustellen, der eine Amplitude von mindestens 1,6 mA und höchstens 2,4 mA umfasst, wobei die Energiequelle ferner dafür eingerichtet ist, einen Gleichstrom während einer Behandlungszeit Tₜ von mindestens 15 Minuten und höchstens 45 Minuten bereitzustellen.

6. Vorrichtung nach einem der vorhergehenden Ansprüche, wobei die erste Elektrode eine Kathode ist und wobei die zweite Elektrode eine Anode ist.

7. Vorrichtung nach einem der vorhergehenden Ansprüche, wobei das Positionierungsmittel ein Stirnband zur Platzierung rings um den Kopf des Patienten und ein Querband umfasst, wobei das Stirnband dafür eingerichtet ist, die erste Elektrode über der rechten supraorbitalen Region zu positionieren, das Querband an dem Stirnband angebracht ist und dafür eingerichtet ist, die zweite Elektrode über der zweiten Region zu positionieren.

8. Vorrichtung nach Anspruch 7, wobei die Energiequelle dafür eingerichtet ist, an einem gekennzeichneten Abschnitt des Stirnbandes, der einem mittigen Abschnitt der Stirn des Patienten entspricht, an dem Stirnband angebracht zu werden.

9. Vorrichtung nach einem der vorhergehenden Ansprüche, wobei die erste und die zweite Elektrode jeweils dafür gestaltet sind, mittels mindestens zweier Druckknöpfe an dem Positionierungsmittel angebracht zu werden, um einen elektrischen Kontakt zwischen einem elektrischen Leiter und der Elektrode bereitzustellen und um ein Drehen der Elektrode zu verhindern.

10. Vorrichtung nach einem der vorhergehenden Ansprüche, wobei die Energiequelle dafür gestaltet ist, mittels mindestens eines Magnets an dem Positionierungsmittel angebracht zu werden.

11. Vorrichtung nach einem der vorhergehenden Ansprüche, wobei die Vorrichtung mindestens zwei elektrische Leiter zum Übertragen von Gleichstrom von der Energiequelle zu den Elektroden umfasst, wobei die Energiequelle dafür gestaltet ist, mittels mindestens zweier Magnete an dem Positionierungsmittel angebracht zu werden und wobei die Magnete außerdem einen elektrischen Kontakt zwischen den elektrischen Leitern und der Energiequelle bereitstellen.

12. Vorrichtung nach einem der vorhergehenden Ansprüche, wobei die Vorrichtung dafür eingerichtet ist, mittels der Elektroden eine Stromdichte von höchstens 100 µA/cm² bereitzustellen.

13. Vorrichtung nach einem der vorhergehenden Ansprüche, wobei die Vorrichtung dafür eingerichtet ist, mittels der Elektroden eine Stromdichte zwischen 50 und 60 µA/cm² bereitzustellen, und wobei die Energiequelle dafür eingerichtet ist, einen Gleichstrom bereitzustellen und den Gleichstrom mit einer Steilheit von mindestens 0,06 mA/s und höchstens 0,6 mA/s zu erhöhen.

14. Vorrichtung nach einem der vorhergehenden Ansprüche, wobei mindestens zwei Magnete dafür gengerichtet sind, eine Drehung der Energiequelle zu verhindern.

15. Vorrichtung nach einem der vorhergehenden Ansprüche, wobei mindestens ein Magnet an dem Positionierungsmittel angeordnet ist, so dass es mittig an einer Stirn des Patienten angelegt ist.

## Revendications

1. Dispositif pour le traitement des troubles de la conscience par stimulation transcrânienne en courant continu, le dispositif comprenant une première électrode et une seconde électrode (4), un moyen de positionnement (1, 2) et une source d'énergie (8), le moyen de positionnement étant :
a. conçu pour positionner la première électrode sur la région supraorbitaire droite (FP2) de la tête d'un patient ;
b. conçu pour positionner la seconde électrode sur une seconde région de la tête du patient, la seconde région correspondant au cortex préfrontal dorso-latéral gauche (F3) ;
c. comprenant au moins un conducteur électrique pour transmettre un courant continu à partir de la source d'énergie aux électrodes,
dans lequel le moyen de positionnement comprend une partie désignée (9) à laquelle la source d'énergie (8) est configurée pour être fixée, moyennant quoi l'emplacement de la partie désignée (9) est configuré pour un positionnement correct de la première et de la seconde électrode lors du positionnement de ladite partie désignée (9) de manière centrale sur le front du patient.

2. Dispositif selon l'une quelconque des revendications précédentes, dans lequel la source d'énergie est conçue pour fournir un courant continu aux électrodes comprenant une amplitude d'au moins 0,5 mA et d'au plus 8 mA.

3. Dispositif selon l'une quelconque des revendications précédentes, dans lequel la source d'énergie est conçue pour fournir un courant continu pendant une durée de traitement Tₜ d'au moins 5 minutes.

4. Dispositif selon l'une quelconque des revendications précédentes, dans lequel la source d'énergie est conçue pour fournir un courant continu pendant une durée de traitement Tₜ d'au plus 180 minutes.

5. Dispositif selon l'une quelconque des revendications précédentes, dans lequel la source d'énergie est conçue pour fournir un courant continu aux électrodes comprenant une amplitude d'au moins 1,6 mA et d'au plus 2,4 mA, dans lequel la source d'énergie est en outre conçue pour fournir un courant continu pendant une durée de traitement Tₜ d'au moins 15 minutes et d'au plus 45 minutes.

6. Dispositif selon l'une quelconque des revendications précédentes, dans lequel la première électrode est une cathode et dans lequel la seconde électrode est une anode.

7. Dispositif selon l'une quelconque des revendications précédentes, dans lequel le moyen de positionnement comprend un bandeau destiné à être placé autour de la tête du patient et une bande transversale, le bandeau étant conçu pour positionner la première électrode sur la région supraorbitaire droite, la bande transversale étant fixée au bandeau et conçue pour positionner la seconde électrode sur la seconde région.

8. Dispositif selon la revendication 7, dans lequel la source d'énergie est configurée pour être fixée au bandeau sur une partie désignée du bandeau, correspondant à une partie centrale du front du patient.

9. Dispositif selon l'une quelconque des revendications précédentes, dans lequel chacune des première et seconde électrodes est configurée pour être fixée au moyen de positionnement par l'intermédiaire d'au moins deux boutons-pression pour assurer un contact électrique entre un conducteur électrique et l'électrode et pour empêcher la rotation de l'électrode.

10. Dispositif selon l'une quelconque des revendications précédentes, dans lequel la source d'énergie est configurée pour être fixée au moyen de positionnement par l'intermédiaire d'au moins un aimant.

11. Dispositif selon l'une quelconque des revendications précédentes, dans lequel le dispositif comprend au moins deux conducteurs électriques pour transmettre un courant continu à partir de la source d'énergie aux électrodes, dans lequel la source d'énergie est configurée pour être fixée au moyen de positionnement par l'intermédiaire d'au moins deux aimants, et moyennant quoi les aimants assurent également un contact électrique entre les conducteurs électriques et la source d'énergie.

12. Dispositif selon l'une quelconque des revendications précédentes, dans lequel le dispositif est conçu pour fournir par l'intermédiaire desdites électrodes une densité de courant d'au plus 100 µA/cm².

13. Dispositif selon l'une quelconque des revendications précédentes, dans lequel le dispositif est conçu pour fournir par l'intermédiaire desdites électrodes une densité de courant comprise entre 50 et 60 µA/cm², et dans lequel la source d'énergie est conçue pour fournir un courant continu et pour augmenter ledit courant continu avec une pente d'au moins 0,06 mA/s et d'au plus 0,6 mA/s.

14. Dispositif selon l'une quelconque des revendications précédentes, dans lequel au moins deux aimants sont conçus pour empêcher la rotation de la source d'énergie.

15. Dispositif selon l'une quelconque des revendications précédentes, dans lequel au moins un aimant est disposé sur le moyen de positionnement de manière à être monté de manière centrale sur le front du patient.
